# EUROPÄISCHE PATENTSCHRIFT

(11) **EP 0 817 610 B1**
(45) Veröffentlichungstag und Bekanntmachung des Hinweises auf die Patenterteilung: **30.05.2001**
(21) Anmeldenummer: 96904826.3
(22) Anmeldetag: 20.02.1996
(51) Int. Cl.: A61K 9/00

(54) **STERILES TROPFBARES OPHTHALMISCHES GELPRÄPARAT UND VERFAHREN ZU DESSEN HERSTELLUNG**
STERILE OPHTHALMOLOGICAL GEL PREPARATION APPLICABLE IN DROPS AND PROCESS FOR PRODUCING IT
PREPARATION DE GEL OPHTALMIQUE STERILE APPLICABLE EN GOUTTES ET SON PROCEDE DE PRODUCTION

(30) Priorität: 28.03.1995 DE 19511322
(43) Veröffentlichungstag der Anmeldung: 14.01.1998
(73) Patentinhaber: Dr. GERHARD MANN chem.-pharm. Fabrik GmbH, D-13581 Berlin (DE)
(72) Erfinder: BELLMANN, Günther, D-13593 Berlin (DE); CLAUS-HERZ, Gudrun, D-14167 Berlin (DE); REIMER HEVIA, Cornelia, D-14612 Falkensee (DE)
(74) Vertreter: Maiwald, Walter, Dr. Dipl.-Chem.
(86) Internationale Anmeldenummer: EP9600697
(87) Internationale Veröffentlichungsnummer: WO9629984

(56) Entgegenhaltungen:
- EP-A- 0 028 110
- EP-A- 0 376 852
- WO-A-95/05163
- US-A- 4 966 773

## Beschreibung

Die Erfindung betrifft ein steriles tropfbares ophthalmisches Gelpräparat, insbesondere zur Verwendung als künstliche Tränenflüssigkeit und zur Behandlung von "trockenem Auge", sowie ein Verfahren zur Herstellung eines solchen Gelpräparats.

Es ist bereits seit längerem bekannt, als Ersatz für die natürliche Tränenflüssigkeit sowie zur Behandlung von "trockenem Auge", wäßrige Präparate einzusetzen, die z.B. Filmbildner auf der Basis von Polyvinylalkohol (PVOH), Polyvinylpyrrolidon (PVP), Cellulose-Derivaten oder Dextran enthalten. Eine Übersicht findet sich beispielsweise bei Sucker, Fuchs und Speiser, Pharmazeutische Technologie, 1978. Es handelt sich hierbei durchweg um Systeme, die nur eine flüssige Phase aufweisen, typischerweise eine wäßrige Lösung weiterer Komponenten. Soweit schwer oder nicht wasserlösliche Komponenten vorhanden sind, sind diese als Feststoffe in feinstverteilter Form in der wäßrigen Phase suspendiert.

Bei halbsynthetischen Präparaten, wie z.B. solchen auf Cellulose-Derivatbasis oder Dextranbasis, ist die Herstellung deshalb recht aufwendig, weil solche Stoffe nur schwer frei von unlöslichen Anteilen erhältlich sind. Um bei Anwendung daraus bereiteter Lösungen Reizungen aufgrund mechanischer Effekte zu vermeiden, muß also ein sehr erheblicher Aufwand getrieben werden. Des weiteren lassen sich solche Zubereitungen nur schwer sterilisieren, da sie nur schwer oder gar nicht durch Filtration von Keimen befreit werden können und sich auch nicht ohne Qualitätsverluste autoklavieren lassen. Bei Cellulose-Derivaten führt die Autoklavierung zu irreversibler Viskositätsverminderung. Bei Dextran führt die Autoklavierung zu teilweiser Zersetzung.

Auch Polyvinyalkohol läßt sich nur dann durch Erhitzen sterilisieren, wenn eine sehr reine verseifte Form eingesetzt wird, denn sonst depolymerisiert die PVOH-Komponente. Da sowohl PVOH als auch PVP nur eine relativ geringe Verdickungswirkung zeigen, müssen große Anteile dieser Komponenten eingesetzt werden, wenn geeignete Viskositäten erreicht werden sollen. Dies führt zu einer hohen Stoffbelastung der ophthalmologischen Zubereitungen, bei PVOH z.B. zu Gehalten von u.U. mehr als 10%.

Beispielsweise aus DE 34 40 352 und DE 43 03 818, wie auch aus US 5,252,318 ist es bereits bekannt, Polyacrylsäure und deren Derivate wie beispielsweise Carbopol® 940 (B.F. Goodrich Company) als Gelbasis für sterile tropfbare Augengele vorzusehen. In US 5,441,732, veröffentlicht nach dem Prioritätsdatum der vorliegenden Anmeldung, ist eine Kombination zweier spezieller Gelkomponenten beschrieben, von denen eine wärmeabhängig und die andere pH-abhängig geliert. Als wärmeempfindliches Gelpolymer werden verschiedene Cellulose-Derivate beschrieben, während als pH-abhängig gelierendes Polymer Polyacrylate, insbesondere vernetzte Polyacrylate erwähnt werden. Die Gelbildung soll anscheinend (durch pH-Änderung) im Auge erfolgen. Diese Polymergemische sollen zusammen mit organischen Ölen eingesetzt werden, in denen sich eine Vielzahl von Wirkstoffen lösen lassen. Vitamin A wird dabei nicht erwähnt.

Die US 5,209,927 betrifft einphasige ophthalmische Zusammensetzungen mit besonderen rheologischen Eigenschaften zur Anwendung als lang einwirkende künstliche Träne.

Im Hinblick auf Verträglichkeit, Reizlosigkeit und Lagerstabilität sind sehr hohe Anforderungen an ophthalmische Zubereitungen zu stellen, die sich als Filmbildner bzw. Schmiermittel eignen sollen. Insbesondere, wenn die ophthalmischen Zubereitungen über längere Zeiträume verabreicht werden müssen, was z.B. bei "trockenem Auge" regelmäßig der Fall ist, wird bereits ein vorübergehendes Augenbrennen als sehr störend empfunden, und ist damit einer Dauerbehandlung abträglich. Es darf verständlicherweise nicht einmal gelegentlich bei der Anwendung solcher Mittel zu Sehstörungen, zu verschwommenem Sehen oder anderen akuten Irritationen kommen.

Bei der Behandlung von "trockenem Auge" hat sich die Verabreichung von Vitamin A, meist in Form des Vitamin A-palmitats bewährt. So sind, nach dem Prioritätsdatum der vorliegenden Anmeldung, im Handel tropfbare Gelpräparate auf Carbopol-Basis angeboten worden, die Vitamin A-palmitat neben anderen üblichen Bestandteilen enthalten. Es handelt sich um einphasige Gele mit einer kontinuierlichen wäßrigen Flüssigphase, ohne flüssige hydrophobe zweite Phase. Diese Produkte haben (bei einer Überdosierung der Vitamin A-Komponente um z.B. 40%) eine Lebensdauer von etwa einem Jahr. Der Vitamin A-Gehalt nimmt, wie Versuche gezeigt haben, in sechs Monaten um etwa 20% ab, so daß bei 40%-iger Überdosierung nach einem Jahr der ausgewiesene Mindestgehalt noch gewährleistet ist.

Die natürliche Tränenflüssigkeit umfaßt u.a. eine fettartige Komponente, enthaltend Triglyceride und Phospholipide. Es ist bereits versucht worden (WO 94/05298), in synthetischer Tränenflüssigkeit Triglyceride einzusetzen, was aber nur unter Verwendung eines Emulgators und in Form einer Emulsion für die topische Applikation am Auge möglich war. Das hat den Nachteil, daß erwünschte Anteile restlicher natürlicher Tränenflüssigkeit des Auges durch den Emulgatoranteil zerstört werden.

Es ist eine wesentliche Aufgabe der Erfindung, ophthalmische Präparate, insbesondere Gelpräparate zu schaffen, die eine erheblich verbesserte Lagerbeständigkeit aufweisen, insbesondere, wenn sie empfindliche Substanzen wie Vitamin A und dessen Derivate enthalten.

Es ist eine weitere wesentliche Aufgabe der Erfindung, solche Präparate so auszugestalten, daß sie, insbesondere bei erhöhter Lagerfähigkeit, keine akuten Irritationen und Sehstörungen auslösen.

Es ist eine weitere sehr wesentliche Aufgabe der Erfindung, solche Präparate so auszugestalten, daß sie auch eine wiederkehrende Anwendung über einen langen Zeitraum und eine verlängerte Verweildauer im Auge nach jeder einzelnen Anwendung ermöglichen, ohne daß es zu Unverträglichkeiten, Augenreizungen und anderen Irritationen kommt.

Die Erfindung beruht auf mehreren überraschenden Feststellungen.

Zum einen hat es sich überraschenderweise erwiesen, daß sich die relativ schnelle Zersetzung von Vitamin A und seinen Derivaten, insbesondere Vitamin A-palmitat, in künstlicher Tränenflüssigkeit und Zusammensetzungen zur Behandlungen von "trockenen Auge" wesentlich reduzieren läßt, wenn das Präparat zweikomponentig mit einer flüssigen wäßrigen und einer flüssigen hydrophoben Phase ausgebildet wird. Insbesondere wird es hierbei bevorzugt, wenn das Präparat eine kontinuierliche wäßrige Phase umfaßt, in der eine hydrophobe flüssige Ölphase in Form feinstverteilter Tröpfchen vorliegt. Hierfür gibt es keine einfache Erklärung. Für die Zersetzung des Vitamin A ist am wahrscheinlichsten die Einwirkung von Sauerstoff und/oder Licht verantwortlich und hinsichtlich beider dürfte es keinen wesentlichen Unterschied machen, ob das Präparat eine oder zwei separate flüssige Phasen umfaßt. Eher würde man bei einer Lösung der Vitamin A-Komponente in der hydrophoben Phase eine erhöhte Empfindlichkeit gegenüber Luftsauerstoff und/oder Licht erwarten.

Möglicherweise spielt für solche Präparate der gleichzeitige Einsatz von Antioxidantien, wie insbesondere Vitamin E und seine Derivate, speziell Vitamin E-Acetat, eine Rolle. Jedenfalls wird Vitamin A in erfindungsgemäßen Präparaten vorzugsweise zusammen mit solchen Antioxidantien eingesetzt.

Ein weiterer überraschender Effekt der Erfindung ist, daß sie die Herstellung von Präparaten mit einer wäßrigen und einer flüssigen hydrophoben Phase ermöglicht, die der Zusammensetzung natürlicher Tränenflüssigkeit sehr nahe kommen und insbesondere einen entsprechenden Gehalt an Triglyceriden enthalten, ohne daß gleichzeitig Emulgatoren eingesetzt werden müssen. Dies ist dadurch möglich, daß erfindungsgemäß solche Präparate als tropfbare Gelpräparate ausgelegt werden. Im Gegensatz zu Präparaten ohne Gelbasis lassen sich Triglyceride in den erfindungsgemäßen Gelpräparaten auch über lange Lagerzeiten in Form von feinstverteilten Tröpfchen vorlegen, die auch ohne Emulgatorgehalt im Gel ausreichend stabil sind. Solche Präparate lassen sich unproblematisch steril herstellen und sind hervorragend verträglich.

Die erfindungsgemäßen Präparate, einschließlich der Gelpräparate, verfügen über einen mit der natürlichen Tränenflüssigkeit weitgehend übereinstimmenden Brechungsindex und zeigen darüber hinaus eine lange Verweildauer am Auge.

Die erfindungsgemäßen Präparate verringern anscheinend die Oberflächenspannung des wäßrigen Augengels, was zu einer besseren Verteilung auf der Cornea führt. Auch kann ein suspendierter bzw. in der Ölphase gelöster Wirkstoff gleichmäßiger verteilt werden. Die Benetzbarkeit von Objekten, die auf die Cornea aufgelegt werden, wie z.B. Kontaktschalen oder Frontlinsen ophthalmologischer Geräte, wird verbessert.

Langzeitstudien zeigen, daß die erfindungsgemäßen Zubereitungen in den handelsüblichen 5g-Polyfoil-Kanülentuben nicht nur unter den Bedingungen gemäßigten Klimas (21°C, 45% rF), sondern auch unter Bedingungen mediterran-subtropischen Klimas (26°C, 60% rF) sowie sogar unter sehr heißen und feuchten klimatischen Verhältnissen (31°C, 70 rF) sehr stabil sind und kaum, oder nur geringfügige, Veränderungen im Hinblick auf Vitamin A-Gehalt (sofern vorgesehen), pH-Wert, Osmolalität, Viskosität und Aussehen zeigen.

Grundsätzlich eignen sich als flüssige hydrophobe Komponente der erfindungsgemäßen zweiphasigen Präparate, insbesondere Gele, solche ophthalmologisch akzeptablen organischen Öle, die sich ohne Emulgatorzusatz tröpfchenförmig in wäßriger Phase dispergieren lassen. Beispiele sind Fettsäurederivate wie insbesondere Fettsäureester, Triglyceride und Phthalsäureester. Besonders bevorzugt werden gegenwärtig Triglyceride, insbesondere überwiegend oder ganz aus C₈-C₁₂-Fettsäuren gebildete homogene bzw. gemischte Triglyceride. Insbesondere werden mittelkettige Triglyceride der Art bevorzugt, wie sie in DAB 10 (1993) beschrieben werden. Hierbei handelt es sich beim Säurebestandteil um ein Gemisch mit einem Anteil von mindestens 95% n-Octansäure und n-Decansäure; der Rest wird von kurzkettigeren Fettsäuren gebildet.

Solche mittelkettigen Triglyceride werden auf semisynthetischem Wege aus dem Öl des festen und des trockenen Teils des Endosperms von *Cocos nucifera L.* dargestellt, indem das gewonnene Cocosöl hydrolisiert, die erhaltenen Fettsäuren fraktioniert und sodann wieder verestert werden.

Solche mittelkettigen Triglyceride sind als Grundstoffe für Kosmetika sowie als Hilfsstoffe und Trägersubstanzen für Pharmazeutika, aber auch für hochwertige Nahrungsmittelerzeugnisse, bereits eingesetzt worden. Über Verwendungsmöglichkeiten und Einsatzbreite solcher mittelkettiger Triglyceride in ophthalmischen Zusammensetzungen ist trotz ihrer vorteilhaften Eigenschaften bisher sehr wenig bekannt.

Erfindungsgemäße Präparate, insbesondere auf Gelbasis, mit typischen Gehalten zwischen 0,1 und 3 Gew.-%, insbesondere etwa 0,2 Gew.-% Polyacrylsäure als Gelbildner in der wäßrigen Phase, enthalten typischerweise zwischen 0,5 und 10 Gew.-%, insbesondere etwa 1 Gew.-% solcher mittelkettigen Triglyceride.

Erfindungsgemäße Gelpräparate zeigen vorzugsweise eine Viskosität im Bereich von etwa 2000-6000 mPa·s bei einem pH-Wert von 6-8.

Die erfindungsgemäßen Präparate enthalten vorzugsweise ein Konservierungsmittel, wie insbesondere Cetrimid, Benzalkoniumchlorid oder Thiomersal. Weiterhin wird mit Vorteil vorgesehen, daß solche Gelpräparate wenigstens ein Isotonikum enthalten, wofür insbesondere Sorbitol in Frage kommt.

Der besonders bevorzugte Gehalt an einer Vitamin A-Komponente, insbesondere Vitamin A-palmitat, liegt im Bereich von 500 internationalen Einheiten pro Gramm des erfindungsgemäßen Präparates. Die Vitamin A-Komponente wird besonders bevorzugt mit einem geringen Gehalt von wenigstens einem Antioxidans stabilisiert, wobei Vitamin E bzw. Vitamin E-Acetat besonders bevorzugt werden.

Die Herstellung des erfindungsgemäßen sterilen Präparate, insbesondere Gele, erfolgt in einem mehrstufigen Prozeß. Bei der Gelherstellung wird vorzugsweise eine sterile Polyacrylsäure-suspension in der Weise erhalten, wie in DE 43 03 818 beschrieben ist, also durch Autoklavieren bei etwa 120°C, 1 bar Überdruck und einer Zeitdauer von 20 Minuten. Parallel dazu wird eine wäßrige Lösung zubereitet, die das Konservierungsmittel und das Isotonikum, also vorzugsweise Cetrimid und Sorbitol, enthält. Diese wäßrige Lösung wird unter Verwendung von Stickstoff als Druckgas, anderenfalls eventuell auch einfacher mit Druckluft, zur autoklavierten Polyacrylsäure-suspension steril hinzufiltriert. Anschließend wird vorsichtig mit steriler Natronlauge neutralisiert, was die Gelbildung auslöst. Nach Beendigung der Neutralisation liegt in dem sich bildenden Gel keine freie Base mehr vor. In das sterile Gel wird dann die hydrophobe flüssige Komponente, also vorzugsweise die mittelkettige Triglycerid-Komponente, unter antiseptischen Bedingungen eingearbeitet. Es wird bis zur vollständigen Homogenisierung gerührt. Die Größe der so erzeugten Öltröpfchen in der erfindungsgemäßen Dispersion liegt bei maximal etwa 100 *µ*m und ist im übrigen der Art, wie sie in einer konventionellen Emulsion ohne Zusatz starker Emulgatoren erhalten werden kann.

Das sterile Gel kann jetzt in üblicher Weise konfektioniert werden.

Anderenfalls wird ein Wirkstoff, wie insbesondere Vitamin A-palmitat, dem so hergestellten sterilen Gel zugesetzt, und zwar in der Weise, daß die Vitamin A-Komponente und die vorzugsweise vorhandene sehr viel geringere Menge des Antioxidans in Neutralöl gelöst und dann steril filtriert wird. Die sterile Öllösung wird dann unter Rühren in das Gel eingearbeitet.

In vergleichbarer Weise wird gearbeitet, wenn ein Präparat ohne Gelbasis, also beispielsweise eine Tropflösung hergestellt werden soll.

Die erfindungsgemäß verwendeten Gelbildner sind vorzugsweise Polyacrylsäuren mit einem Molekulargewicht im Bereich von etwa 3-5 Millionen. Besonders bevorzugt werden handelsübliche Produkte, wie Carbopol® -Polymersäuren, wie von B.F. Goodrich Chemicals Co. vertrieben. Besonders bevorzugt wird Carbopol 980 NF® . Die Konzentration im Präparat liegt vorzugsweise im Bereich von etwa 0,2 Gew.-%.

Die zur Gelbildung notwendige Neutralisation wird üblicherweise mit steriler verdünnter Natronlauge durchgeführt, wobei 1-N Natronlauge besonders vorteilhaft ist. Daneben können aber auch andere anorganische Basen bzw. Alkalicarbonate oder aber organische Basen, wie Amine, insbesondere Triethylamin und Diisopropylamin verwendet werden.

Die auf diese Art erhaltenen erfindungsgemäßen Gele zeigen Viskositäten im Bereich von etwa 2000-6000 mpa·s bei 20°C.

Das üblicherweise erfindungsgemäß verwendete Konservierungsmittel, wie beispielsweise Cetrimid, Benzalkoniumchlorid oder Thiomersal, wird in der üblichen Konzentration, also bei Cetrimid mit etwa 0,01 Gew.-% eingesetzt. Die Isotonisierungsmittel, also beispielsweise mehrwertige Alkohole, wie Mannitol, Dextrose, Glycerin, Propylenglycol oder, besonders bevorzugt, Sorbitol, werden mit Konzentrationen üblicher Art eingesetzt. Bei Sorbitol wird besonders vorteilhaft eine Konzentration von etwa 4,85 Gew.-% verwendet.

Die Erfindung wird im folgenden anhand zweier Beispiele näher erläutert:

### Beispiel 1

In eine Prozeßanlage wird eine homogene Suspension aus ca. 375 kg Wasser für Injektionszwecke mit 2,000 kg Polyacrylsäure (Carbopol 980 NF®) über ein Faserfangfilter mit einer Porenweite von etwa 25-40 *µ*m eingebracht. Die Suspension wird unter Rühren bei 121°C und 1 bar Überdruck für 20 Minuten autoklaviert und anschließend auf Raumtemperatur heruntergekühlt, wobei über das sterilisierte Luftfilter belüftet wird.

Zwischenzeitlich werden in einem geeigneten Ansatzbehälter ca. 469 kg Wasser für Injektionszwecke vorgelegt und darin unter Rühren zuerst 0,100 kg Cetrimid, anschließend 48,510 kg Sorbitol gelöst. Diese Lösung wird über eine dampfsterilisierte Membranfilterkerze mit einer Porenweite von 0,2 *µ*m unter Verwendung von Stickstoff als Druckgas zur bereits autoklavierten Polyacrylsäure-Suspension gegeben. Abschließend wird ggf. mehrfach evakuiert, um entstandenen Schaum zu zerstören.

Unter sterilen Bedingungen werden 0,832 kg Natriumhydroxid in ca. 20 kg Wasser für Injektionszwecke unter Rühren gelöst. Die Natronlauge wird über eine dampfsterilisierte Membranfilterkerze zur Cetrimid/Sorbitol/Polyacrylsäure-Suspension filtriert, woraufhin noch die Restmenge Wasser für Injektionszwecke zugegeben werden. Das sich bildende Gel wird mit einem Homogenisator umgewälzt.

Zum sterilen Gel werden dann mittelkettige Triglyceride über ein Sterilfilter mit einer Porenweite von 0,2 *µ*m eingearbeitet, wobei bis zur vollständigen Homogenisierung gerührt wird. Vom entstehenden Gel wird der pH-Wert bestimmt, der bei 6-8 (bei 20°C) liegen soll. Die Osmolalität der erfindungsgemäßen Zubereitung liegt in einem Bereich von 260-320 mOsm/kg. Das auf diese Art und Weise erhaltene Gel wird unter aseptischen Bedingungen in 5g-Polyfoil-Kanülentuben abgefüllt.

### Beispiel 2

In 9,37g Neutralöl (Myritol 318® ) werden 0,6g Vitamin A-Palmitat und 0,03 Vitamin E-Acetat gelöst.

Die Lösung wird über ein 0,22 µm Filter (Millipore®) steril filtriert.

Zu 990g Gel, hergestellt gemäß Beispiel 1, werden 10g der Neutralöllösung zugesetzt und unter Rühren mit dem Flügelrührer eingearbeitet. Nach 20 Minuten Rühren wird das fertige Produkt in 10g Polyfoil-Tuben abgefüllt. Das Gel entspricht einem Gehalt von 500 internationalen Einheiten Vitamin A pro Gramm Präparat, bei einem 20%-igen Überschuß.

### Vergleichsbeispiel

Ein Carbopolgel wird gemäß Beispiel 1 hergestellt; dabei wird die mittelkettige Triglyceridkomponente jedoch weggelassen.

### Vergleichsversuch

Wie beim Beispiel 2 wird das Gel gemäß dem Vergleichsbeispiel mit einem entsprechenden Gehalt von Vitamin A-Palmitat versehen.

Proben dieses Vitamin A-Präparates ohne Triglyceridkomponente werden zusammen mit entsprechenden Proben gemäß Beispiel 2 unter Standardbedingungen gelagert. Nach insgesamt 6 Monaten Lagerzeit hat sich der Vitamin A-Gehalt bei den Vergleichsproben ohne Triglyceridkomponente um insgesamt 20% verringert.

Bei den Proben gemäß Beispiel 2 ist der Vitamin A-Gehalt innerhalb der Meßgenauigkeit unverändert.

## Patentansprüche

1. Steriles tropfbares ophthalmisches Präparat, insbesondere Gelpräparat,
**dadurch gekennzeichnet,** daß das Präparat eine zweiphasige, eine flüssige wäßrige Phase und eine flüssige hydrophobe Phase umfassende Trägerflüssigkeit bzw. Gelbasis enthält und frei von Emulgatoren ist.

2. Präparat nach Anspruch 1,
**dadurch gekennzeichnet,** daß das Präparat die wäßrige Phase als kontinuierliche Phase und die hydrophobe Phase als darin dispergierte Tröpfchen enthält.

3. Präparat nach Anspruch 1 oder 2,
**dadurch gekennzeichnet,** daß die wäßrige Phase wenigstens eine polymere gelbildende Komponente, insbesondere eine Polyacrylsäure oder ein polymeres Acrylsäurederivat in einer Menge enthält, die zur Ausbildung der Gel-Eigenschaften des Präparats ausreicht.

4. Präparat nach Anspruch 3,
**dadurch gekennzeichnet,** daß das Präparat zwischen 0,1 und 3 Gew.-%, insbesondere etwa 0,2 Gew.-% Polyacrylsäure enthält.

5. Präparat nach einem der Ansprüche 1-4,
**dadurch gekennzeichnet,** daß die hydrophobe Phase ein ophthalmologisch akzeptables, organisches Öl, insbesondere ein ohne Emulgatorzusatz tröpfchenförmig in wäßriger Phase bleibend dispergierbares Öl umfaßt.

6. Präparat nach Anspruch 5,
**dadurch gekennzeichnet,** daß das Öl wenigstens ein Fettsäurederivat, ein Triglycerid und/oder einen Phthalsäureester umfaßt.

7. Präparat nach Anspruch 6
**dadurch gekennzeichnet,** daß das Öl ein Triglycerid, insbesondere ein mittelkettiges Triglycerid und besonders bevorzugt ein überwiegend oder ganz aus C₈-C₁₂-Fettsäuren gebildetes Misch-Triglycerid umfaßt.

8. Präparat nach einem der Ansprüche 6-7,
**dadurch gekennzeichnet,** daß das Präparat zwischen 0,5 und 10 Gew.-%, insbesondere etwa 1 Gew.-% mittelkettige Triglyceride enthält.

9. Präparat nach einem der vorstehenden Ansprüche,
**dadurch gekennzeichnet,** daß das Präparat eine Viskosität im Bereich von etwa 2000-6000 mPa·s aufweist.

10. Präparat nach einem der vorstehenden Ansprüche,
**dadurch gekennzeichnet,** daß das Präparat einen pH-Wert von 6-8 aufweist.

11. Präparat nach einem der vorstehenden Ansprüche,
**dadurch gekennzeichnet,** daß das Präparat wenigstens einen ophthalmischen Wirkstoff in therapeutisch wirksamer Menge enthält.

12. Präparat nach Anspruch 11,
**dadurch gekennzeichnet,** daß das Präparat eine Vitamin A-Komponente, insbesondere Vitamin A-Palmitat, besonders bevorzugt in Kombination mit einem Antioxidans wie Vitamin E oder Vitamin E-Acetat, enthält.

13. Präparat nach einem der vorstehenden Ansprüche,
**dadurch gekennzeichnet,** daß das Präparat ein Konservierungsmittel und gegebenenfalls ein Isotonikum enthält.

14. Präparat nach Anspruch 13,
**dadurch gekennzeichnet,** daß das Konservierungsmittel Cetrimid, Benzalkoniumchlorid oder Thiomersal ist und gegebenenfalls das Isotonikum Sorbitol ist.

15. Verfahren zur Herstellung eines Präparates nach einem der Ansprüche 1-14,
**dadurch gekennzeichnet,** daß eine flüssige hydrophobe Phase, insbesondere eine mittelkettige Triglyceridkomponente, homogen in einer kontinuierlichen wäßrigen Phase, insbesondere einem zuvor hergestellten sterilen Hydrogel, dispergiert wird.

16. Verfahren nach Anspruch 15,
**dadurch gekennzeichnet,** daß eine Polyacrylsäurekomponente eingesetzt wird, die durch Neutralisation der Carboxylgruppen mit einer geeigneten Base, insbesondere Natronlauge, zur Gelbildung umgesetzt wird.

17. Verfahren nach Anspruch 16,
**dadurch gekennzeichnet,** daß dem sterilen Präparat der Wirkstoff unter aseptischen Bedingungen zugesetzt und homogen eingemischt wird.

## Claims

1. Sterile ophthalmic preparation applicable in drop form, in particular a gel preparation,
characterized in that the preparation comprises a two-phase carrier liquid or gel base, respectively, comprising a liquid aqueous phase and a liquid hydrophobic phase, and the preparation is free of emulsifiers.

2. Preparation according to claim 1,
characterized in that the preparation comprises the aqueous phase as a continuous phase and the hydrophobic phase as droplets dispersed therein.

3. Preparation according to claim 1 or 2,
characterized in that the aqueous phase comprises at least one polymeric gel-forming component, in particular a polyacrylic acid or a polymeric acrylic acid derivative, in an amount sufficient to provide the gel-like properties of the preparation.

4. Preparation according to claim 3,
characterized in that the preparation comprises between 0.1 and 3 weight percent, in particular about 0.2 weight percent, of polyacrylic acid.

5. Preparation according to any one of claims 1-4,
characterized in that the hydrophobic phase comprises an ophthalmologically acceptable organic oil, in particular a dispersible oil, which is capable of remaining in the aqueous phase in the form of droplets without addition of emulsifier.

6. Preparation according to claim 5,
characterized in that the oil comprises at least one fatty acid derivative, a triglyceride and/or a phthalic acid ester.

7. Preparation according to claim 6,
characterized in that the oil comprises a triglyceride, in particular a medium chain triglyceride and especially preferred a mixed triglyceride formed predominantly or in total from C₈-C₁₂ fatty acids.

8. Preparation according to claim 6 or 7,
characterized in that the preparation comprises between 0.5 and 10 weight percent, in particular about 1 weight percent, medium chain triglycerides.

9. Preparation according to any one of the previous claims,
characterized in that the preparation has a viscosity in the range of about 2000-6000 mPa·s.

10. Preparation according to any one of the previous claims,
characterized in that the preparation has a pH value of 6-8.

11. Preparation according to any one of the previous claims,
characterized in that the preparation comprises at least one ophthalmic drug in a therapeutically effective amount.

12. Preparation according to claim 11,
characterized in that the preparation comprises a vitamin A component, in particular vitamin A palmitate, especially preferred in combination with an antioxidant like vitamin E or vitamin E acetate.

13. Preparation according to any one of the previous claims,
characterized in that the preparation comprises a preservative and optionally an isotonic agent.

14. Preparation according to claim 13,
characterized in that the preservative is cetrimid, benzalconium chloride or thiomersal and optionally the isotonic agent is sorbitol.

15. Method for the manufacture of a preparation according to any one of claims 1-14,
characterized in that a liquid hydrophobic phase, in particular a medium chain triglyceride component, is dispersed homogeneously in a continuous aqueous phase, in particular in a previously prepared sterile hydrogel.

16. Method according to claim 15,
characterized in that a polyacrylic acid component is used, which is reacted by neutralization of the carboxyl groups with a suitable base, in particular sodium hydroxide, in order to form the gel.

17. Method according to claim 16,
characterized in that the drug is added to the sterile preparation under aseptic conditions and is admixed homogeneously.

## Revendications

1. Préparation ophtalmique stérile applicable en gouttes, en particulier préparation sous forme de gel, caractérisée en ce que la préparation contient un liquide véhicule et/ou une base de gel à deux phases comprenant une phase liquide aqueuse et une phase liquide hydrophobe, et en ce qu'elle est exempte d'émulsifiants.

2. Préparation selon la revendication 1, caractérisée en ce que la préparation contient la phase aqueuse sous forme de phase continue et la phase hydrophobe sous forme de gouttelettes dispersées dans celle-ci.

3. Préparation selon la revendication 1 ou 2, caractérisé en ce que la phase aqueuse comprend au moins un composant polymère formant un gel, en particulier un acide polyacrylique ou un dérivé polymère d'acide acrylique dans une quantité suffisante pour obtenir les propriétés du gel de la préparation.

4. Préparation selon la revendication 3, caractérisée en ce que la préparation contient entre 0,1 et 3 pourcent en poids, en particulier approximativement 0,2 pourcent en poids d'acide polyacrylique.

5. Préparation selon l'une quelconque des revendications 1 à 4, caractérisée en ce que la phase hydrophobe comprend une huile organique acceptable du point de vue ophtalmologique, en particulier une huile dispersible restant sous forme de gouttelettes en phase aqueuse, sans addition d'émulsifiant.

6. Préparation selon la revendication 5, caractérisée en ce que l'huile comprend au moins un dérivé d'acide gras, un triglycéride et/ou un ester d'acide phtalique.

7. Préparation selon la revendication 6, caractérisée en ce que l'huile comprend un triglycéride, en particulier un triglycéride à chaîne moyenne, et de manière particulièrement préférée un triglycéride mixte formé principalement ou totalement d'acides gras C₈ à C₁₂.

8. Préparation selon l'une ou l'autre des revendications 6 et 7, caractérisée en ce que la préparation contient entre 0,5 et 10 pourcent en poids, en particulier approximativement 1 pourcent en poids de triglycéride à chaîne moyenne.

9. Préparation selon l'une quelconque des revendications précédentes, caractérisée en ce que la préparation présente une viscosité dans la plage d'approximativement 2000 à 6000 mPa·s.

10. Préparation selon l'une quelconque des revendications précédentes, caractérisée en ce que la préparation présente un pH d'une valeur de 6 à 8.

11. Préparation selon l'une quelconque des revendications précédentes, caractérisée en ce que la préparation contient au moins une substance ophtalmique en quantité thérapeutique active.

12. Préparation selon la revendication 11, caractérisée en ce que la préparation contient un composant de vitamine A, en particulier un palmitate de vitamine A, de manière particulièrement préférée en combinaison avec un antioxydant tel que la vitamine E ou l'acétate de vitamine E.

13. Préparation selon l'une quelconque des revendications précédentes, caractérisée en ce que la préparation contient un agent conservateur et le cas échéant un isotonique.

14. Préparation selon la revendication 13, caractérisée en ce que l'agent conservateur est de la cétrimide, du chlorure de benzalkonium ou du thiomersal, et en ce que le cas échéant l'isotonique est du sorbitol.

15. Procédé de production d'une préparation selon l'une quelconque des revendications 1 à 14, caractérisé en ce qu'une phase hydrophobe liquide, en particulier un composant triglycéride à chaîne moyenne, est dispersé de manière homogène dans une phase aqueuse continue, en particulier dans un hydrogel stérile produit auparavant.

16. Procédé selon la revendication 15, caractérisé en ce que l'on met en oeuvre un composant d'acide polyacrylique qui est transformé pour la formation de gel, par neutralisation des groupes carboxyles avec une base appropriée, en particulier de la soude caustique.

17. Procédé selon la revendication 16, caractérisé en ce que le principe actif est ajouté à la préparation stérile dans des conditions aseptiques et mélangé de manière homogène.
